# EUROPÄISCHE PATENTSCHRIFT

(11) **EP 0 902 690 B1**
(45) Veröffentlichungstag und Bekanntmachung des Hinweises auf die Patenterteilung: **19.12.2001**
(21) Anmeldenummer: 97925035.4
(22) Anmeldetag: 03.06.1997
(51) Int. Cl.: A61M 1/10

(54) **BALLONKATHETER**
BALLOON CATHETER
CATHETER A BALLONNET

(30) Priorität: 04.06.1996 DE 19622335
(43) Veröffentlichungstag der Anmeldung: 24.03.1999
(73) Patentinhaber: Völker, Wolfram, 69469 Weinheim (DE)
(72) Erfinder: Völker, Wolfram, 69469 Weinheim (DE)
(74) Vertreter: Selting, Günther, Dipl.-Ing.
(86) Internationale Anmeldenummer: EP9702864
(87) Internationale Veröffentlichungsnummer: WO9746270

(56) Entgegenhaltungen:
- WO-A-93/05712
- US-A- 4 753 221
- US-A- 5 163 910
- US-A- 5 628 719

## Beschreibung

Die Erfindung betrifft einen Ballonkatheter, der in ein Blutgefäß eingeführt werden kann, um beispielsweise Gefäßverengungen aufzuweiten oder einen Teil der Wand des Blutgefäßes vorübergehend vom Kontakt mit dem Blutstrom abzusperren.

Bekannt sind Ballonkatheter mit einem Katheterschlauch, der am distalen Ende einen rohrförmigen Ballonträger aufweist, auf welchem ein ringförmiger Ballon sitzt. Der Ballon steht über ein durch den Katheterschlauch hindurchgehendes Lumen mit einer extrakorporalen Druckquelle in Verbindung. Durch Einleiten einer Flüssigkeit unter Druck wird der Ballon aufgeweitet. Dabei erfolgt normalerweise ein Totalverschluß des Blutgefäßes, mit der Folge einer Mangelversorgung (Ischämie) der nachfolgenden Organregionen. Um diese zu vermeiden, werden Perfusionskatheter (Durchblutungskatheter) benutzt. Zu diesen gehören Autoperfusionskatheter (DE 92 07 395 U1), die einen durch den Ballonträger hindurchgehenden Durchlaß aufweisen, so daß eine Blutströmung durch das Innere des ringförmigen Ballons erfolgt, sowie aktive Perfusionssysteme, bei denen mit Hilfe einer externen Hochdruckpumpe, die an das distale Katheterende angeschlossen wird, eine kontinuierliche Perfusion durch das Katheterlumen erfolgt. Im Gegensatz zu Autoperfusionskathetern wird bei aktiven Perfusionssystemen ein bestimmter Druck aktiv aufgebaut, der unabhängig von dem aktuellen Blutdruck des Patienten ist. Hierzu muß dem Patienten jedoch arterialisiertes Blut entnommen werden, das extrakorporal in das Lumen des Katheterschlauchs gepumpt wird. Zudem sind sehr hohe Pumpendrücke (bis ca. 12 bar) aufgrund der großen Druckverluste im kleinlumigen Katheterschlauch erforderlich, um ca. 40 bis 60 ml/min. zu fördern.

Aus US-A-4 969 865 ist eine intravasale Blutpumpe bei Herzunterstützung bekannt. Diese Blutpumpe kann durch ein Blutgefäß hindurch in das Herz eingeführt werden. Sie weist ein längliches Rohr auf, in dem ein schraubenförmiger Propeller angeordnet ist, der durch eine flexible Welle angetrieben wird. Diese Pumpe saugt axial an und sie weist am entgegengesetzten Austrittsende Löcher für das radiale Abströmen auf. Im Betrieb wird die Blutpumpe in der Öffnung der Herzklappe plaziert, wobei das Ansaugende sich in einem der Herzventrikel befindet, während das Abströmende in der Aorta angeordnet ist.

US-A-5 163 910 beschreibt ein, eine Pumpanordnung enthaltener, Ballonkatheter. Der Oberbegriff des Anspruchs 1 entspricht der Veröffentlichung dieses Dokumentes.

Der Erfindung liegt die Aufgabe zugrunde, einen Ballonkatheter zu schaffen, der ein vereinfachtes aktives Perfusionssystem bildet.

Die Lösung dieser Aufgabe erfolgt erfindungsgemäß mit den im Patentanspruch 1 angegebenen Merkmalen.

Bei dem erfindungsgemäßen Ballonkatheter ist eine Pumpe im Zuge des Katheters angeordnet, und zwar proximal von dem Ballonträger. Die Pumpe saugt seitlich über entsprechende Öffnungen Blut aus dem Blutgefäß an und pumpt es axial durch den Ballonträger hindurch. Da die Pumpe unmittelbar vor dem Ballonträger angeordnet ist, ist nur ein relativ geringer Pumpendruck erforderlich. Die Pumpe ist so klein, daß sie direkt im Bereich des Eingriffs, also in der Nähe des Ballons, im Katheter plaziert werden kann, so daß die Länge des zu durchspülenden Lumens kurz ist, wodurch der Druckverlust relativ gering gehalten wird. Die Pumpe hat einen so kleinen Durchmesser, daß sie im Zuge des Katheters in das Blutgefäß eingeführt werden kann, ohne dieses zu versperren. Von einer Behandlung mit Ballonkathetern sind in der Regel kleinere Blutgefäße betroffen. Der maximale Außendurchmesser der Pumpe sollte etwa 1,4 mm nicht übersteigen. Eine derartig kleine Pumpe kann in den Katheter integriert werden, so daß sie unmittelbar vor dem Ballonträger, der durch die Behandlungsstelle des Blutgefäßes hindurchgeht, liegt. Dabei saugt die Pumpe in Strömungsrichtung des Gefäßes aus dem Bereich höheren Drucks Blut über mindestens einen seitlichen Einlaß an und fördert dieses in den Niederdruckbereich jenseits der aufgeblasenen Ballons.

Wegen der erforderlichen Kleinheit der Pumpe muß diese mit relativ hohen Drehzahlen betrieben werden, um die erforderliche Förderleistung von beispielsweise 60 cm³/min. zu erreichen. Die Pumpendrehzahl liegt vorzugsweise im Bereich von 80.000 bis 150.000 U/min.

Der Ballonkatheter wird vorzugsweise für das Dehnen von Gefäßverengungen, speziell im Herzkranzgefäßbereich, in den Halsschlagadern u.dgl., eingesetzt. Es ist aber auch möglich, den Ballonkatheter dazu zu benutzen, einen Drahtkorb (Stent) zur Weitstellung von Gefäßverengungen zu plazieren.

Das Perfusionskatheter der Erfindung kann mehrere hintereinander angeordnete Ballons aufweisen. Die Ballons dichten einen Längenabschnitt des Gefäßes vorübergehend ab, um in diesem Bereich einen Bypaß annähen zu können. Das Blut strömt weiterhin durch den rohrförmigen Ballonträger, während der abgedichtete Gefäßabschnitt für operative Eingriffe zur Verfügung steht.

Die Erfindung schafft einen aktiven Perfusionskatheter mit integrierter Pumpe. Dieser entspricht in seiner Handhabung weitgehend den passiven Autoperfusionskathetern, ist in seiner Leistung aber den aktiven Perfusionssystemen vergleichbar. Ein weiterer Vorteil besteht darin, daß der Ballonträger mit einem sehr geringen Außendurchmesser von etwa 1 mm realisiert werden kann. Hierdurch ist in fast allen Fällen, selbst bei starken Stenosen, gewährleistet, daß eine Plazierung des Ballonbereichs in der Stenose erfolgen kann.

Vorzugsweise ist die Pumpe von einer flexiblen Welle angetrieben, die mit einem am proximalen Ende des Katheterschlauchs vorgesehenen Motor verbunden ist. Solche Pumpen, die nur aus einem Pumpengehäuse und dem darin rotierend angeordneten Flügelrad bestehen, können mit einem sehr geringen Außendurchmesser von etwa 1,5 mm gefertigt werden. Generell ist es im Rahmen der Erfindung aber auch möglich, einen Pumpenmotor in den Katheter, in direkter Nähe zur Pumpe, zu integrieren, wobei der Motor dann das Pumpenrad unmittelbar antreibt.

Um einen Ballonkatheter in einem Blutgefäß zu plazieren, wird in der Regel ein Führungsdraht eingebracht, über den der Katheter anschließend geschoben wird. Eine derartige Verlegung mit einem Führungsdraht ist auch bei dem erfindungsgemäßen Ballonkatheter vorgesehen. Hierbei muß der Führungsdraht allerdings durch die Pumpe hindurchgeführt werden. Das Flügelrad der Pumpe oder die Pumpe selbst ist demnach so ausgebildet, daß es den Durchgang eines Führungsdrahtes, der einen Durchmesser von etwa 0,3 mm hat, ermöglicht. Zu berücksichtigen ist, daß die Pumpe erst in Betrieb genommen wird, nachdem der Führungsdraht hinreichend weit aus dem unmittelbaren Pumpenbereich herausgezogen wurde. Während der Verlegung des Katheters ist es hinnehmbar, wenn der Führungsdraht das Pumpenrad blockiert.

Im folgenden werden unter Bezugnahme auf die Zeichnungen Ausführungsbeispiele der Erfindung näher erläutert.

Es zeigen:
- Fig. 1: einen Längsschnitt durch den distalen Endbereich des Ballonkatheters,
- Fig. 2: den Ballonkatheter nach Fig. 1 mit eingeschobenem Führungsdraht, wobei die Pumpe eine Monorail-Führung bildet, und
- Fig. 3: ein Ausführungsbeispiel, bei dem der Führungsdraht koaxial durch die Pumpe hindurchgeht.

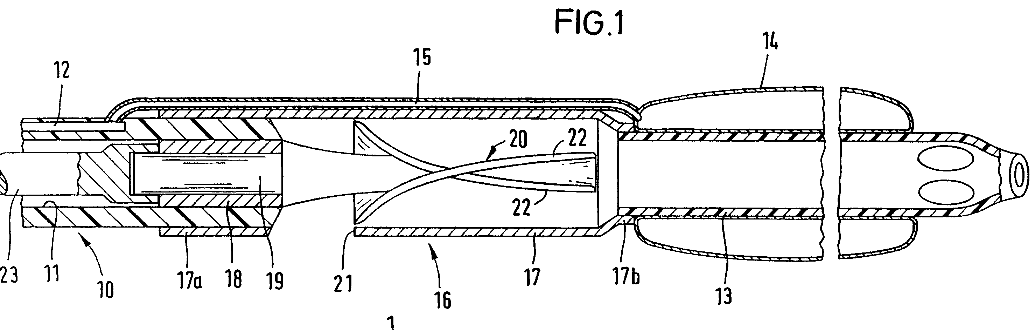

Der Ballonkatheter 1 nach Fig. 1 weist einen langgestreckten Katheterschlauch 10 auf, dessen Länge etwa 1 m beträgt und dessen Durchmesser ca. 1,3 mm beträgt. Der flexible Katheterschlauch 10 weist ein zentrisches Schlauchlumen 11 mit einem Durchmesser von ca. 0,8 mm auf. Ferner verläuft durch die Wand des Katheterschlauchs 10 ein weiteres Lumen 12 zur Zuleitung von Druckfluid zu dem Ballon 14.

An der Katheterspitze befindet sich der rohrförmige Ballonträger 13 mit einem Innendurchmesser von ca. 0,8 mm und einem Außendurchmesser von ca. 1,0 mm. Dieser Ballonträger 13 ist von mindestens einem Ballon 14 umgeben. Der Ballon 14 steht über eine Leitung 15 mit dem Lumen 12 des Katheterschlauchs 10 in Verbindung. Das vordere Ende des Ballonträgers 12 ist verjüngt ausgebildet und weist eine axiale und mehrere seitliche Blutaustrittsöffnungen auf.

Zwischen dem Katheterschlauch 10 und dem Ballonträger 13 befindet sich die Pumpe 16. Diese weist ein rohrförmiges Pumpengehäuse 17 mit einem Außendurchmesser von ca. 1,5 mm auf. Das proximale Ende 17a des Pumpengehäuses ist an dem Ende des Katheterschlauchs 10 befestigt und das distale Ende 17b ist mit dem Ballonträger 13 verbunden. Das Pumpengehäuse 17 besteht aus Metall und es enthält ein Lager 18, in dem die Welle 19 des Pumpenrades 20 gelagert ist. Das Ende des Katheterschlauchs 10 ist zwischen dem Lager 18 und dem Pumpengehäuse 17 eingepaßt. Unmittelbar im Anschluß an das Ende des Katheterschlauchs 10 sind an dem Gehäuse 17 seitliche Einlässe 21 vorgesehen, durch die Blut radial in das Pumpengehäuse 17 eintreten kann. In dem Bereich, im dem sich das Pumpenrad 20 befindet, ist das Gehäuse 17 umfangsmäßig geschlossen. Das Pumpenrad 20 weist mehrere Flügel 22 auf, die schraubenförmig um die Achse herum angeordnet sind und sich über einen Winkelbereich von mindestens 150° erstrecken. Die axiale Länge des Pumpenrades 20 beträgt ca. das Doppelte des maximalen Durchmessers dieses Pumpenrades. Das Pumpenrad 20 endet unmittelbar vor dem Ballonträger 13.

Die Welle 19 des Pumpenrades 20 ist mit einer flexiblen Welle 23 verbunden, die im Innern des Lumens 11 des Katheterschlauchs 10 verläuft und am proximalen Ende mit einem externen Elektromotor verbunden ist, der die Welle mit einer Drehzahl in der Größenordnung von 100.000 U/min. antreibt.

Während der Ballonbereich des Katheters beispielsweise in den Bereich einer Stenose eingeführt und der Ballon 14 unter Druck gesetzt und dadurch aufgeweitet wurde, fördert die Pumpe 16 Blut von den Einlässen 21 durch den Ballonträger 13 hindurch. Das distale Ende des Ballonträgers 13 ist offen oder mit Öffnungen versehen, so daß das Blut dort aus dem Ballonträger 13 in das Blutgefäß hinein austreten kann.

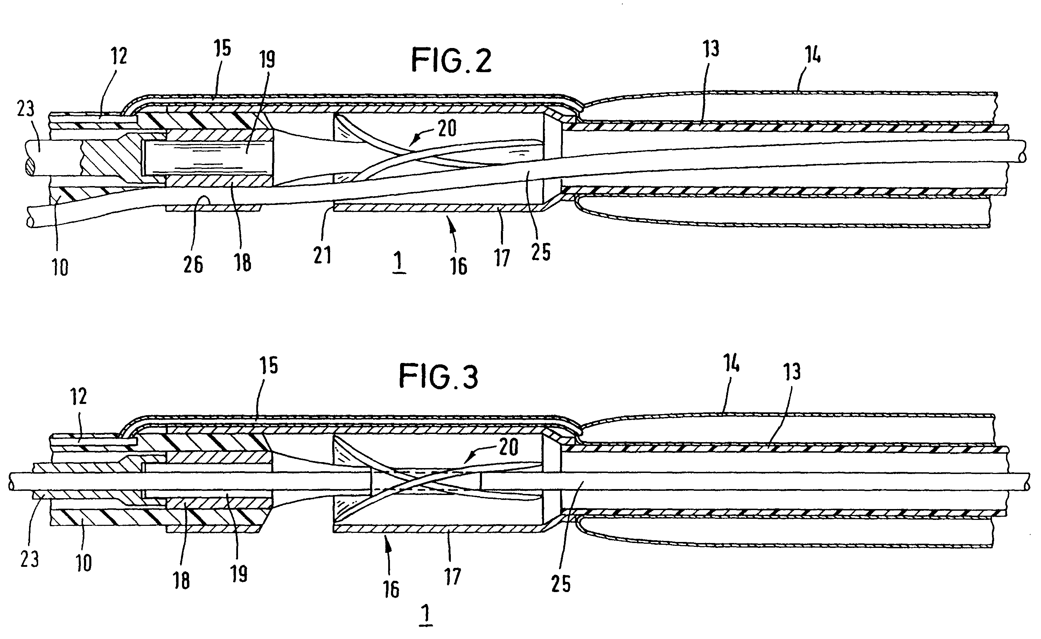

In Fig. 2 wird das Ballonkatheter gemäß der Erfindung mit einem zusätzlichen Führungsdraht 25 geschildert, der zunächst in dem Blutgefäß plaziert wird und über den dann der Katheter geschoben wird. Dieser flexible Führungsdraht 25 verläuft generell außerhalb des Katheterschlauchs 10. Am distalen Ende des Katheterschlauchs und in einem Teil des Pumpengehäuses 17, vorzugsweise auf den letzten 10 cm des Katheterschlauchs 10 befindet sich ein längslaufender Kanal 26, der einen Führungsbereich (Monorail) bildet, durch den der Führungsdraht 25 in das Pumpengehäuse 17 geleitet wird. Der Durchmesser des Führungsdrahtes beträgt ca. 0,3 mm, so daß der Führungsdraht durch das Pumpenrad 20 zwischen dessen Flügeln hindurch verlaufen kann. Während der Führungsdraht 25 sich im Pumpengehäuse befindet, erfolgt keine Rotation des Pumpenrades. Nach dem Plazieren des Katheters wird der Führungsdraht für die Behandlung mindestens an dem Pumpengehäuse herausgezogen. Dabei ist der Kanal 26 so plaziert und das Flügelrad 20 so gestaltet, daß der Führungsdraht 25 jederzeit nach erfolgter Behandlung erneut über das stehende Flügelrad 20 in Richtung des Ballons durch den Ballonträger 13 hindurch geschoben werden kann, um das System erneut mit Hilfe des Führungsdrahtes 25 in einer veränderten Position zu plazieren, ohne den Ballonkatheter 1 hierfür völlig zurückziehen zu müssen.

Bei dem Ausführungsbeispiel von Fig. 3 verläuft der Führungsdraht 25 koaxial durch die flexible Welle 23, die Welle 19 und das Flügelrad 20 hindurch. Diese Teile haben entsprechende Axialkanäle, um über den Führungsdraht geschoben werden zu können (Over-the-Wire-Technique). Während des Betriebes der Pumpe kann der Führungsdraht zurückgezogen werden, um den Durchfluß durch den Ballonträger 13 zu verbessern. Nach dem Zurückziehen des Führungsdrahtes ist ein erneutes Vorschieben möglich, wenn die Plazierung des Katheters verändert werden soll. Für die Funktion der Pumpe ist dies allerdings nicht erforderlich.

Befinden sich auf dem Ballonträger mehrere Ballons, so kann für jeden der Ballons eine hydraulische Zuleitung 15 über die Pumpe 16 hinweg vorgesehen werden. Dennoch sollte der Katheter in seinem dicksten Bereich, also im Bereich der Pumpe 16, speziell für den Einsatz im Koronarbereich (Karotinen), nicht wesentlich über 1,5 mm Durchmesser hinausgehen, da sonst eine Plazierung des Katheters über einen standardmäßigen Einführkatheter mit 2 mm Innendurchmesser nicht mehr möglich wäre. Ferner ist es erforderlich, daß die Länge des Pumpengehäuses 17 ca. 6 mm nicht übersteigt.

Die zuvor beschriebenen Ausführungsbeispiele beziehen sich auf einen Ballonkatheter, der eine nach dem Niederdruckprinzip arbeitenden Pumpe 16 enthält, die in der Nähe des Ballonträgers, also am Ende des Katheterschlauchs, angeordnet ist. Hierbei braucht die Pumpe nur einen relativ geringen Druck zu erzeugen. Im Rahmen der Erfindung ist es aber auch möglich, eine intravasale Pumpe im Zuge des Katheterschlauchs anzuordnen, die einen größeren Abstand von dem Ballonträger hat. Eine solche Pumpe muß einen höheren Pumpendruck liefern, weil im Zuge des Katheterschlauchs ein Druckabfall stattfindet.

## Patentansprüche

1. Ballonkatheter (1) mit einem Katheterschlauch (10), und einem mit dem Katheterschlauch verbundenen rohrförmigen Ballonträger (13), der mindestens einen aufweitbaren Ballon (14) trägt,
einer im Bereich des Katheterschlauchs (10) oder zwischen diesem und dem Ballonträger (13) angeordneten intravasalen Pumpe (16), die durch mindestens einen seitlichen Einlaß (21) Blut ansaugt und axial in den Ballonträger (13) fördert,
**dadurch gekennzeichnet**
**daß** die Pumpe (16) in den Katheter integriert ist und einen längslaufenden Führungskanal (26) für einen längs des Katheterschlauchs (10) verlaufenden Führungsdraht (25) aufweist.

2. Ballonkatheter nach Anspruch 1, **dadurch gekennzeichnet, daß** der Führungsdraht (25) weitgehend außerhalb des Katheterschlauchs (10) verläuft und der Führungskanal (26) in das Innere der Pumpe (16) hineinführt.

3. Ballonkatheter nach Anspruch 1 oder 2, **dadurch gekennzeichnet, daß** sich der Führungskanal (26) vom hinteren Ende des Katheterschlauchs bis zum Beginn des Pumpenrades (20) erstreckt.

4. Ballonkatheter nach Anspruch 1, **dadurch gekennzeichnet, daß** der Führungsdraht (25) im Katheterschlauch (10) und koaxial durch die Pumpe (16) verläuft.

5. Ballonkatheter nach einem der Ansprüche 1-4, **dadurch gekennzeichnet, daß** die Pumpe (16) von einer flexiblen Welle (23) angetrieben ist, die mit einem am proximalen Ende des Katheterschlauchs (10) vorgesehenen Motor verbunden ist.

6. Ballonkatheter nach einem der Ansprüche 1-4, **dadurch gekennzeichnet, daß** die Pumpe (16) von einem im Verlauf des Katheterschlauchs angeordneten Motor angetrieben ist.

7. Ballonkatheter nach einem der Ansprüche 1-6, **dadurch gekennzeichnet, daß** die Pumpe (16) ein Pumpenrad (20) mindestens einen Flügel (22) aufweist, dessen Länge mindestens so groß ist wie der maximale Durchmesser des Pumpenrades (20).

8. Ballonkatheter nach einem der Ansprüche 1-7, **dadurch gekennzeichnet, daß** die seitlichen Einlässe (21) wenigstens teilweise bei aufgeblasenem Ballon (14) einen Abstand zur Gefäßwand aufweisen.

9. Ballonkatheter nach Anspruch 8, **dadurch gekennzeichnet, daß** der Abstand zwischen den seitlichen Einlässen (21) und dem Ballon (14) maximal der Länge der Flügel des Pumpenrades (20) entspricht.

10. Ballonkatheter, insbesondere nach einem der Ansprüche 1-9, **dadurch gekennzeichnet, daß** auf dem Ballonträger mindestens zwei Ballons mit gegenseitigem Abstand angeordnet sind.

## Claims

1. A balloon catheter (1) comprising a catheter hose (10) and a tubular balloon support (13) connected to the catheter hose and carrying at least one expandable balloon (14),
a pump (16) disposed in the area of the catheter hose (10) or between the same and the balloon support (13), the pump drawing blood through at least one lateral inlet (21) and delivering it axially into the balloon support (13)
**characterized in**
**that** the pump (16) is integrated in the catheter and comprises a longitudinal guide channel (26) for a guide wire (25) extending along the catheter hose (10).

2. The balloon catheter of claim 1, **characterized in that** the guide wire (25) extends substantially outside the catheter hose (10) and that the guide channel (26) leads into the pump (16).

3. The balloon catheter of claim 1 or 2, **characterized in that** the guide channel (26) extends from the rear end of the catheter hose (about 10 cm) up to the beginning (of the blades) of the impeller wheel (20).

4. The balloon catheter of claim 1, **characterized in that** the guide wire (25) extends within the catheter hose (10) and coaxially through the pump (16).

5. The balloon catheter of one of claims 1 - 4, **characterized in that** the pump (16) is driven by a flexible shaft (23) connected with a motor arranged at the proximal end of the catheter hose (10).

6. The balloon catheter of one of claims 1-5, **characterized in that** the pump (16) is driven by a motor arranged along the length of the catheter hose.

7. The balloon catheter of one of claims 1-6, **characterized in that** the pump (16) comprises an impeller wheel (20) with at least one blade (22), the length of which is at least equal to the maximum diameter of the impeller wheel (20).

8. The balloon catheter of one of claims 1-7, **characterized in that** the lateral inlets (21) are at least partly spaced from the vessel wall when the balloon (14) is inflated.

9. The balloon catheter of claim 8, **characterized in that** the distance between the lateral inlets (21) and the balloon (14) is at most equal to the length of the blades of the impeller wheel (20).

10. The balloon catheter of one of claims 1 - 9, **characterized in that** at least two mutually spaced balloons are arranged on the balloon support.

## Revendications

1. Cathéter à ballon (1) comportant un tuyau souple (10) de cathéter et un porte-ballon tubulaire (13) relié au tuyau souple de cathéter et portant au moins un ballon élargissable (14),
une pompe intravasale (16), placée dans la zone du tuyau souple (10) de cathéter ou entre celui-ci et le porte- ballon (13), pompe qui aspire du sang par au moins une entrée latérale (21) et le refoule axialement dans le porte-ballon (13),
**caractérisé en ce que**
la pompe (16) est intégrée dans le cathéter et comporte un canal de guidage longitudinal (26) pour un fil métallique de guidage (25) disposé le long du tuyau souple(10) de cathéter.

2. Cathéter à ballon selon la revendication 1, **caractérisé en ce que** le fil métallique de guidage (25) est disposé, dans une large mesure, en dehors du tuyau souple (10) de cathéter et que le canal de guidage (26) aboutit à l'intérieur de la pompe (16).

3. Cathéter à ballon selon la revendication 1 ou 2, **caractérisé en ce que** le canal de guidage (26) s'étend de l'extrémité arrière du tuyau souple de cathéter jusqu'au début de la roue (20) de pompe.

4. Cathéter à ballon selon la revendication 1, **caractérisé en ce que** le fil métallique de guidage (25) est disposé dans le tuyau (10) de cathéter et traverse la pompe (16) coaxialement.

5. Cathéter à ballon selon l'une des revendications 1 à 4, **caractérisé en ce que** la pompe (16) est entraînée par un arbre flexible (23), qui est relié à un moteur prévu à l'extrémité proximale du tuyau souple (10) de cathéter.

6. Cathéter à ballon selon l'une des revendications 1 à 4, **caractérisé en ce que** la pompe (16) est entraînée par un moteur placé dans le tracé du tuyau souple de cathéter.

7. Cathéter à ballon selon l'une des revendications 1 à 6, **caractérisé en ce que** la pompe (16) comporte une roue (20) de pompe avec au moins une ailette (22), dont la longueur est au moins aussi grande que le diamètre maximal de la roue (20) de pompe.

8. Cathéter à ballon selon l'une des revendications 1 à 7, **caractérisé en ce que** les entrées latérales (21) présentent, au moins partiellement, lorsque le ballon (14) est gonflé, un écartement par rapport à la paroi du vaisseau.

9. Cathéter à ballon selon la revendication 8, **caractérisé en ce que** la distance entre les entrées latérales (21) et le ballon (14) correspond au maximum à la longueur des ailettes de la roue (20) de pompe.

10. Cathéter à ballon, en particulier selon l'une des revendications 1 à 9, **caractérisé en ce qu'**au moins deux ballons sont placés à distance l'un de l'autre sur le porte-ballon.
